(19) Europäisches Patentamt
European Patent Office
Office européen des brevets

(11) EP 4 640 749 A1

(12) **EUROPEAN PATENT APPLICATION**
published in accordance with Art. 153(4) EPC

(43) Date of publication:
29.10.2025 Bulletin 2025/44

(21) Application number: 23906935.4

(22) Date of filing: 15.12.2023

(51) International Patent Classification (IPC):
$C08J\ 3/12^{(2006.01)}$    $A61K\ 8/00^{(2006.01)}$
$A61K\ 8/25^{(2006.01)}$    $A61K\ 8/73^{(2006.01)}$
$C08G\ 77/42^{(2006.01)}$    $C08K\ 3/36^{(2006.01)}$
$C08L\ 1/00^{(2006.01)}$

(52) Cooperative Patent Classification (CPC):
A61K 8/00; A61K 8/25; A61K 8/73; C08G 77/42;
C08J 3/12; C08K 3/36; C08L 1/00

(86) International application number:
PCT/JP2023/045128

(87) International publication number:
WO 2024/135573 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.12.2022 JP 2022206379

(71) Applicant: Nissin Chemical Industry Co., Ltd.
Fukui 915-0802 (JP)

(72) Inventors:
• MITSUISHI, Hirofumi
Echizen-shi, Fukui 915-0802 (JP)
• WATANABE, Kentaro
Echizen-shi, Fukui 915-0802 (JP)

(74) Representative: Schicker, Silvia
Wuesthoff & Wuesthoff
Patentanwälte und Rechtsanwalt PartG mbB
Schweigerstraße 2
81541 München (DE)

(54) **COMPOSITE PARTICLES, METHOD FOR PRODUCING COMPOSITE PARTICLES, AND COSMETIC**

(57) The present invention is a composite particle including: a particle of cellulose or a cellulose derivative; and silica adhered to a surface of the particle, wherein the silica is a polymerized product of a tetraalkoxysilane in an amount of 1 to 60 parts by mass with respect to 100 parts by mass of the particle. Thus, fine particle excellent in biodegradability and tactile sensation can be provided.

[FIG. 1]

10 μm

## Description

TECHNICAL FIELD

The present invention relates to a cellulose-silica composite particle, its alkoxy-modified composite particle, a method for producing the same, and a cosmetic.

BACKGROUND ART

Conventionally, various polymer fine particles have been proposed according to applications. For example, fine particles are incorporated in cosmetics for various purposes. The purpose of incorporating fine particles in cosmetics is to improve the spreadability of cosmetics, to change the tactile sensation, to impart a wrinkle blurring effect, to improve the slipperiness of foundation, or the like.

Particularly, fine particles with high sphericity have excellent tactile sensation, and a light scattering (soft focus) effect can be obtained depending on their physical properties and shape. Such fine particles, when used for foundation, etc., fill and smoothen the skin irregularities and scatter light in various directions, and thus an effect (soft focus effect) can be expected that makes wrinkles, etc., less noticeable.

For the intended purpose and effect of such a cosmetic, the fine particles formulated in the cosmetic are required to have a narrow particle size distribution and high sphericity; and as such fine particles, fine particles made of synthetic polymers, such as polyamide such as nylon 12, polymethyl methacrylate (PMMA), and polystyrene (PS) have been proposed.

However, since these fine particles made of synthetic polymers are light-weight with a specific gravity of 1 or less and have a too small particle size, the fine particles easily float on water and are not removed by the sewage treatment facility in some cases, and may flow into the river and further into the sea. For this reason, a problem is that the oceans, etc., may be contaminated with the fine particles made of synthetic polymers.

In addition, since the fine particles made of synthetic polymers have the characteristics of adsorbing trace amounts of chemical pollutants in the environment, there is a concern that they may cause various adverse effects: for example, plankton or fish may swallow the fine particles that have adsorbed chemical pollutants, potentially causing adverse effects on humans.

Such concerns have prompted efforts to replace fine particles of synthetic polymers used in various applications with biodegradable particles.

A representative biodegradable resin is cellulose. Cellulose is superior in that it can be obtained from natural materials such as wood and cotton, which do not compete with food and feed. For this reason, it is desirable to develop fine particles containing cellulose.

In addition, synthetic resin powders mainly made of silicone are used in hair cosmetics, makeup cosmetics, sunscreens, and many other cosmetics as important components to form a uniform coating on the skin and hair surface to provide moisture and smoothness, and to impart water repellency and water resistance. For example, Patent Document 1 discloses silicone fine particles, which have soft tactile sensation and are non-aggregating and excellent in dispersion. These are therefore suitable for formulation as a cosmetic; however, there is a need to develop better resin powders.

Patent Document 2 discloses formulating, into a cosmetic composition, particles obtained by surface-treating cellulose acetate particles with a lipophilicity-imparting agent containing a silicone-based component. However, the method includes adhering the lipophilicity-imparting agent to cellulose acetate particles by wet treatment method, which requires the use of organic solvent such as n-hexane; and accordingly, the development of composite particles in a more environmentally friendly aqueous system is demanded.

CITATION LIST

PATENT LITERATURE

Patent Document 1: JP H07-196815 A
Patent Document 2: WO 2020/188698 A1

SUMMARY OF INVENTION

TECHNICAL PROBLEM

In view of the above circumstances, the present invention aims to provide fine particle with excellent biodegradability and excellent tactile sensation.

SOLUTION TO PROBLEM

To solve the above problems, the present invention provides a composite particle comprising: a particle of cellulose or a cellulose derivative; and silica adhered to a surface of the particle, wherein the silica is a polymerized product of a tetraalkoxysilane in an amount of 1 to 60 parts by mass with respect to 100 parts by mass of the particle.

Such composite particle is a fine particle with excellent biodegradability and tactile sensation, in which a biodegradable cellulose particle is coated with silica that imparts properties such as lubricity and water repellency.

In addition, it is also preferable that the particle of cellulose or a cellulose derivative has a volume average particle diameter of 1 to 300 μm.

With such a volume average particle diameter, the composite particle of the present invention is more excellent in tactile sensation and is more suitable for use in cosmetic.

In addition, it is also preferable that the tetraalkoxysilane is tetramethoxysilane.

With such a configuration, it is possible to more reliably obtain silica in a state adhered to the cellulose particle by subjecting the tetraalkoxysilane to hydrolysis and condensation reactions.

In addition, it is also preferable that the composite particle of the present invention forms a coating film containing the composite particle and having a coefficient of static friction of less than 0.50.

With such a composite particle, it is possible to obtain a cosmetic with excellent slipperiness.

In addition, it is preferable that the composite particle is alkoxy-modified.

With such a configuration, it is possible to obtain a particle with water repellency.

The present invention further provides a composite particle comprising: a particle of cellulose or a cellulose derivative; and silica adhered to a surface of the particle, wherein the particle is coated with silica in a specific surface area of 50% or more.

Such composite particle is a fine particle with excellent biodegradability and tactile sensation, in which a biodegradable cellulose particle is coated with silica that imparts properties such as lubricity and water repellency.

In addition, it is also preferable that the particle of cellulose or a cellulose derivative has a volume average particle diameter of 1 to 300 μm.

With such a volume average particle diameter, the composite particle of the present invention is more excellent in tactile sensation and is more suitable for use in cosmetics.

In addition, it is preferable that the silica is a polymerized product of a tetraalkoxysilane.

With such a configuration, it is possible to obtain silica in a state adhered to the cellulose particle by subjecting the tetraalkoxysilane to hydrolysis and condensation reactions.

In addition, it is also preferable that the tetraalkoxysilane is tetramethoxysilane.

With such a configuration, it is possible to more reliably obtain silica in a state adhered to the cellulose particle by subjecting the tetraalkoxysilane to hydrolysis and condensation reactions.

In addition, it is also preferable that the composite particle of the present invention forms a coating film containing the composite particle and having a coefficient of static friction of less than 0.50.

With such a composite particle, it is possible to obtain a cosmetic with excellent slipperiness.

In addition, it is preferable that the composite particle is alkoxy-modified.

With such a configuration, it is possible to obtain a particle with water repellency.

The present invention also provides a method for producing a composite particle comprising a particle of cellulose or a cellulose derivative and silica adhered to a surface of the particle, the method comprising the step of:

(1) in the presence of (A) a particle of cellulose or a cellulose derivative, water, and an alkali, hydrolyzing and condensing 1 to 60 parts by mass of (B) a tetraalkoxysilane with respect to 100 parts by mass of the (A) particle to form silica, and adhering the silica to a surface of the (A) particle to obtain the composite particle.

Thus, the composite particle of the present invention can be produced by water-based reaction.

In this case, the method may further comprise the step of (2) removing water after step (1).

Thus, the composite particle of the present invention can be obtained in powder form.

The present invention further provides a cosmetic comprising the above composite particle.

The composite particle of the present invention can be suitably used in cosmetic.

In this case, it is preferable that the cosmetic contains the composite particle in an amount of 1 to 50 mass% based on a total amount of the cosmetic.

With such a blending amount, the cosmetic of the present invention can sufficiently exhibit the effects of the composite particle of the present invention.

ADVANTAGEOUS EFFECTS OF INVENTION

When formulated and used in a cosmetic, the composite particle of the present invention can impart properties such as lubricity, a soft tactile sensation, and water repellency to the cosmetic. Therefore, the composite particle of the present invention can be formulated into many cosmetics such as hair cosmetics, makeup cosmetics, sunscreens, and the like. Moreover, the particle is biodegradable and therefore environmentally friendly.

BRIEF DESCRIPTION OF DRAWINGS

FIG. 1 is an electron micrograph showing the surface of the composite particle obtained in Example 3;
FIG. 2(a) is an electron micrograph (a) showing the surface of the composite particle obtained in Example 3;
FIG. 2(b) is an image (b), which is a superimposed image of the element mapping image of silicon and the element mapping image of carbon corresponding to the image shown in FIG. 2(a);
FIG. 2(c) is an image (c) showing only the silicon signal corresponding to the image shown in FIG. 2(b);
FIG. 3 is an electron micrograph showing the surface of the composite particle obtained in Comparative Example 4.

DESCRIPTION OF EMBODIMENTS

As described above, there has been a demand for the development of fine particles with excellent biodegradability and tactile sensation.
As a result of extensive studies to achieve the above-described object, the present inventors have developed a composite particle in which a cellulose particle is coated with silica, thereby completing the present invention.
That is, the present invention provides a composite particle comprising a particle of cellulose or a cellulose derivative; and silica adhered to a surface of the particle, wherein the silica is a polymerized product of a tetraalkoxysilane in an amount of 1 to 60 parts by mass with respect to 100 parts by mass of the particle, and a method for producing the same, as well as a cosmetic containing the composite particle. Furthermore, the present invention provides a composite particle obtained by alkoxy-modifying the above-mentioned composite particle, as well as a method for producing the same, and a cosmetic containing the alkoxy-modified composite particle.
The present invention will be described in detail hereinbelow, but is not limited thereto.

[Composite Particle]

The present invention is a composite particle comprising: a particle of cellulose or a cellulose derivative; and silica adhered to a surface of the particle, wherein the silica is a polymerized product of a tetraalkoxysilane in an amount of 1 to 60 parts by mass with respect to 100 parts by mass of the particle. Hereinafter, it will be described in detail.

(A) Particle of cellulose or cellulose derivative

The particle of cellulose or a cellulose derivative (hereinafter collectively referred to as cellulose particle) is a particle containing cellulose. The cellulose derivative particle may be a cellulose derivative particle such as cellulose acetate or cellulose acetate propionate. The shape of the cellulose particle is not limited to spherical and may be shapeless; however, spherical cellulose particle is preferred.
Preferably, the volume average particle diameter of the cellulose particle is 1 to 300 $\mu$m. More preferably, the volume average particle diameter is 1 to 150 $\mu$m, and still more preferably, 1 to 50 $\mu$m. More specifically, examples of the volume average particle diameter include 3 $\mu$m, 5 $\mu$m, 7 $\mu$m, 10 $\mu$m, 15 $\mu$m, 20 $\mu$m, and 45 $\mu$m. Here, the volume average particle diameter of the cellulose particle is the 50% cumulative volume particle diameter determined from the data obtained by measurement using a laser diffraction particle size analyzer (for example, "SALD2100" manufactured by SHIMADZU CORPORATION).
The bulk density of the cellulose particle is preferably from 0.4 to 1.0 $g/cm^3$. When the bulk density is 1.0 $g/cm^3$ or less, the composite particle can impart a light feel when formulated into a cosmetic, and also exhibits preferable oil absorption. The bulk density can be calculated by placing the sample in a container of about 200 mL, measuring the weight after removing the surplus sample on the top surface of the container, and calculating by the following formula.

Bulk density $(g/cm^3)$ = Sample weight (g)/Container volume $(cm^3)$

For example, a Powder Tester PT-X (manufactured by HOSOKAWA MICRON CORPORATION) can be used as a bulk density measurement device.

Preferably, the angle of repose of the cellulose particle is 60° or less. When the angle of repose is 60° or less, there is no risk of imparting a sticky tactile sensation, which is preferable. The angle of repose can be measured by dropping approximately 10 g of a sample from a funnel having a diameter of 6 mm to form a mound on a receiving dish below, and measuring the angle formed between the slope of the mound and the horizontal plane. For example, a Powder Tester PT-X (manufactured by HOSOKAWA MICRON CORPORATION) can be used as an angle of repose measurement device.

The cellulose particle or cellulose derivative may be produced, for example, by dissolving cellulose acetate in an organic solvent to prepare a cellulose acetate solution, suspending the solution in water to prepare a suspension in which cellulose acetate particles are dispersed in water, removing the organic solvent from the suspension, and then saponifying the cellulose acetate particle such that the amount of acetic acid relative to the mass of cellulose is 0.5 ppm or less. After the cellulose acetate particle is saponified to obtain a cellulose particle, water may be further removed by solid-liquid separation, and the cellulose particle may be dried.

Examples of commercially available cellulose particle that can be used include BELLOCEA (registered trademark, produced by DAICEL CORPORATION, cellulose acetate), Viscopearl (registered trademark, produced by Rengo Co., Ltd.), and ART PEARL NC-400 and NC-800 (produced by Negami Chemical Industrial Co., Ltd.).

(B) Silica

The composite particle of the present invention is formed by allowing silica to adhere to the surface of the above-described cellulose particle. The silica is a polymerized product of a tetraalkoxysilane. The tetraalkoxysilane is represented by the following formula.

$$Si(OR)_4$$

(In the formula, R represents a hydrogen atom or an alkyl group having 1 to 4 carbon atoms, and is most preferably a methyl group.)

One tetraalkoxysilane may be used alone or two or more thereof may be used in combination.

Preferred examples of the above tetraalkoxysilane include tetramethoxysilane, tetraethoxysilane, tetrapropoxysilane, and tetrabutoxysilane. Among them, tetramethoxysilane, in which R is a methyl group, is particularly preferred.

In the composite particle of the present invention, the adhered silica is a polymerized product obtained by the hydrolysis and condensation reaction of tetraalkoxysilane, and has a structure containing $SiO_2$ units; however it may also contain an alkoxy group derived from the raw material tetraalkoxysilane or a silanol group that has not undergone condensation.

[Production Method]

The present invention provides a method for producing a composite particle comprising a particle of cellulose or a cellulose derivative and silica adhered to a surface of the particle, the method comprising the step of: (1) in the presence of (A) a particle of cellulose or a cellulose derivative, water, and an alkali, hydrolyzing and condensing 1 to 60 parts by mass of (B) a tetraalkoxysilane with respect to 100 parts by mass of the (A) particle to form silica, and adhering the silica to a surface of the (A) particle to obtain the composite particle. Subsequently, the method may further include the step of (2) removing water. Here, the silica is a polymer containing $SiO_2$ units. The production method according to the present invention will now be described in detail.

Step (1) of the production method according to the present invention comprises hydrolyzing and condensing (B) a tetraalkoxysilane in the presence of (A) a cellulose particle, water, and an alkali to form silica. The silica obtained through the hydrolysis and condensation reactions can be obtained in a state adhered to the cellulose particle.

The alkali functions as a catalyst for hydrolyzing and condensing the (B) tetraalkoxysilane, or as a catalyst for condensing the same. One alkali may be used alone or two or more thereof may be used in combination. The alkali may be added as is, or may be added in the form of an alkaline aqueous solution. Furthermore, the alkali may be added to the aqueous dispersion containing the cellulose particle and water before the addition of the tetraalkoxysilane, or may be added after the addition of the tetraalkoxysilane.

The amount of the alkali to be added is preferably such that the pH of the aqueous dispersion containing the cellulose particle and water falls within the range of 9.0 to 12.0, more preferably within the range of 9.5 to 11.5. When the pH is within the above range, the hydrolysis and condensation reactions of the tetraalkoxysilane can sufficiently proceed, and the resulting silica can sufficiently adhere to the surface of the cellulose particle.

The alkali is not particularly limited as long as it promotes the hydrolysis and condensation reactions of the (B) tetraalkoxysilane. Examples include alkali metal hydroxides such as potassium hydroxide, sodium hydroxide, and lithium hydroxide; alkaline earth metal hydroxides such as calcium hydroxide and barium hydroxide; alkali metal carbonates such as potassium carbonate and sodium carbonate; ammonia; tetraalkylammonium hydroxides such as tetramethylammonium hydroxide and tetraethylammonium hydroxide; and amines such as monomethylamine, dimethylamine, diethylamine, trimethylamine, triethanolamine, and ethylenediamine, any of which may be used. Among them, ammonia is most suitable, since it can be easily removed by volatilization from the powder of the resulting cellulose particle. An aqueous ammonia solution commercially available can be used as the ammonia.

The amount of the (B) tetraalkoxysilane to be added is in the range of 1 to 60 parts by mass, preferably 5 to 50 parts by mass, and more preferably 15 to 40 parts by mass, with respect to 100 parts by mass of the (A) cellulose particle. When the amount of the (B) tetraalkoxysilane is less than 1 part by mass, the effect of silica is not exhibited. When the amount thereof is more than 60 parts by mass, silica not coating the cellulose particle or an aggregated particle may be generated to deteriorate the texture, producing a hard or grainy texture when a cosmetic is formulated therefrom.

The (B) tetraalkoxysilane is preferably added under stirring at 50 to 500 rpm using a conventional blender such as a propeller-type or flat-blade impeller.

In addition, a surfactant or a water-soluble polymer may be added to the aqueous dispersion of cellulose for the purpose of controlling the adhesion of the silica to the surface of the cellulose particle.

The surfactant to be added to the aqueous dispersion is not particularly limited. Examples thereof include nonionic surfactants, anionic surfactants, cationic surfactants, and amphoteric surfactants. Two or more types of surfactants may be used in combination. When a surfactant is added, the amount thereof is preferably in the range of 0.01 to 10 parts by mass with respect to 100 parts by mass of the (A) cellulose particle.

Examples of the nonionic surfactant include polyoxyethylene alkyl ethers, polyoxyethylene polyoxypropylene alkyl ethers, polyoxyethylene alkylphenyl ethers, polyethylene glycol fatty acid esters, sorbitan fatty acid esters, polyoxyethylene sorbitan fatty acid esters, polyoxyethylene sorbitol fatty acid esters, glycerin fatty acid esters, polyoxyethylene glycerin fatty acid esters, polyglycerin fatty acid esters, propylene glycol fatty acid esters, polyoxyethylene castor oil, polyoxyethylene hydrogenated castor oil, polyoxyethylene fatty acid amides, polyoxyethylene modified organopolysiloxanes, and polyoxyethylene polyoxypropylene modified organopolysiloxanes.

Examples of the anionic surfactant include alkyl sulfate ester salts, polyoxyethylene alkyl ether sulfate ester salts, polyoxyethylene alkylphenyl ether sulfate ester salts, fatty acid alkylolamide sulfate ester salts, alkylbenzene sulfonate salts, $\alpha$-sulfo fatty acid ester salts, alkyl naphthalene sulfonate salts, alkyl diphenyl ether disulfosuccinate salts, fatty acid salts, polyoxyethylene alkyl ether carboxylate salts, N-acylamino acid salts, monoalkyl phosphate ester salts, dialkyl phosphate ester salts, and polyoxyethylene alkyl ether phosphate ester salts.

Examples of the cationic surfactant include alkyltrimethylammonium salts, dialkyldimethylammonium salts, polyoxyethylene alkyl dimethylammonium salts, di(polyoxyethylene) alkyl methylammonium salts, tri(polyoxyethylene) alkylammonium salts, alkylbenzyldimethylammonium salts, alkylpyridinium salts, monoalkylamine salts, and monoalkylamideamine salts.

Examples of the amphoteric surfactant include alkyl dimethylamine oxides, alkyl dimethyl carboxybetaines, alkylamidopropyl dimethyl carboxybetaines, alkyl hydroxy sulfobetaines, and alkyl carboxymethyl hydroxyethyl imidazolinium betaines.

The water-soluble polymer to be added to the aqueous dispersion is not particularly limited. Examples thereof include nonionic water-soluble polymers, anionic water-soluble polymers, cationic water-soluble polymers, and amphoteric water-soluble polymers. One water-soluble polymer may be used alone or two or more thereof may be used in combination. When a water-soluble polymer is added, the amount thereof is preferably in the range of 0.01 to 10 parts by mass with respect to 100 parts by mass of the (A) cellulose particle.

Examples of the nonionic water-soluble polymer include a copolymer of vinyl alcohol and vinyl acetate, a polymer of acrylamide, a polymer of vinylpyrrolidone, a copolymer of vinylpyrrolidone and vinyl acetate, polyethylene glycol, a polymer of isopropylacrylamide, a polymer of methyl vinyl ether, starch, methylcellulose, hydroxyethylcellulose, hydroxypropylcellulose, guar gum, and xanthan gum.

Examples of the anionic water-soluble polymer compound include a polymer of sodium acrylate, a copolymer of sodium acrylate and sodium maleate, a copolymer of sodium acrylate and acrylamide, a polymer of sodium styrenesulfonate, a copolymer of sodium polyisoprenesulfonate and styrene, a polymer of sodium naphthalenesulfonate, carboxymethyl starch, phosphate starch, carboxymethylcellulose, sodium alginate, gum arabic, carrageenan, sodium chondroitin sulfate, and sodium hyaluronate.

Examples of the cationic water-soluble polymer include a polymer of dimethyldiallylammonium chloride, a polymer of vinylimidazoline, a polymer of methylvinylimidazolium chloride, a polymer of ethyltrimethylammonium chloride acrylate, a polymer of ethyltrimethylammonium chloride methacrylate, a polymer of acrylamidepropyltrimethylammonium chloride, a polymer of methacrylamidepropyltrimethylammonium chloride, a polymer of epichlorohydrin/dimethylamine, a polymer of ethyleneimine, a quaternized product of a polymer of ethyleneimine, a polymer of allylamine hydrochloride, polylysine, cationic starch, cationized cellulose, chitosan, and derivatives thereof obtained by copolymerizing these with monomers having nonionic or anionic groups.

Examples of the amphoteric water-soluble polymer include a copolymer of ethyltrimethylammonium chloride acrylate, acrylic acid, and acrylamide; a copolymer of ethyltrimethylammonium chloride methacrylate, acrylic acid, and acrylamide; and a Hofmann degradation product of a polymer of acrylamide.

One embodiment of the production method according to the present invention involves adding an alkali to an aqueous dispersion containing the (A) cellulose particle and water, followed by adding the (B) tetraalkoxysilane. In this case, the tetraalkoxysilane may be added at once, but is preferably added gradually over a period of time. When the tetraalkoxysilane is added, the reaction temperature is preferably 0 to 60°C, and more preferably in the range of 0 to 40°C. When the temperature is within the above range, silica can be favorably adhered to the surface of the cellulose particle. Thereafter, the hydrolysis reaction of the tetraalkoxysilane proceeds. Stirring is continued at least until the tetraalkoxysilane dissolves in water. At this time, to promote the hydrolysis reaction, a small amount of alkali is preferably added. The stirring is continued after the tetraalkoxysilane is added and until the hydrolysis and condensation reaction of the tetraalkoxysilane is completed. To complete the hydrolysis, the reaction may be performed at room temperature or under heating at about 40 to 100°C, or an alkali may be additionally added as appropriate.

In another embodiment of the production method according to the present invention, the tetraalkoxysilane may be added before adding an alkali. In this case, it is preferable to first add tetraalkoxysilane to water. The tetraalkoxysilane may be added to water at once, or may be added gradually over a period of time. Alternatively, water may be added to the tetraalkoxysilane, or water and tetraalkoxysilane may be charged at the same time in a vessel to mix them. The temperature is not particularly limited when tetraalkoxysilane is added to water, and may be, for example, in the range of 0 to 100°C. Thereafter, the hydrolysis reaction of the tetraalkoxysilane proceeds. Stirring is continued at least until the tetraalkoxysilane dissolves in water. At this time, to promote the hydrolysis reaction, it is preferable to add a small amount of alkali.

Subsequently, an aqueous dispersion containing the cellulose particle is added to the solution obtained above, followed by adding an alkali. When an alkali is added, the condensation reaction of the hydrolyzate of tetraalkoxysilane proceeds to produce silica. However, at this time, it is necessary to stop stirring before silica is produced, or stir very slowly. If the reaction solution is flowing at high speed during silica formation, silica cannot favorably adhere to the cellulose particle.

During the condensation reaction, the temperature is preferably 0 to 60°C, and more preferably in the range of 0 to 40°C. When the temperature is within the above range, silica can be favorably adhered to the cellulose particle. Until silica is formed (i.e., silica is adhered to the surface of the cellulose particle), the reaction solution is preferably allowed to stand or being stirred very slowly. The standing time is preferably in the range of 10 minutes to 24 hours. Thereafter, an alkali may be further added or heating may be performed at 40 to 100°C to complete the condensation reaction. Furthermore, normal stirring may be additionally performed.

In the production method according to the present invention, the step (2) is a step of removing water, in which, after adhering silica on the surface of the cellulose particle, water is volatilized and removed.

Water may be volatilized and removed under atmospheric pressure or under reduced pressure (0 to 200°C), or by heating (preferably at 100 to 200°C) under atmospheric pressure or under reduced pressure. Examples thereof include a method of allowing the dispersion to stand under heating to remove water. As a pretreatment for this operation, the dispersion may be concentrated by filtration separation, centrifugation, and decantation, etc., or the dispersion may be washed with water or water-soluble alcohol if necessary.

When the powder of the composite particle obtained by volatilizing and removing water is aggregated, the aggregate may be crushed by a crusher such as a jet mill, a ball mill, and a hammer mill.

In the composite particle obtained by the above method, the cellulose particle is covered with silica. The covering silica can be confirmed by infrared absorption spectrum. In addition, the covering state can be confirmed by observing the surface with an electron micrograph. In particular, the cellulose particle is preferably covered with silica in a specific surface area of 30 to 95%, preferably 40 to 90%, and more preferably 50 to 70%.

When the cellulose particle has a surface coverage of less than 30%, the effect of siloxane is not exhibited. Also, when the coverage is 50% or more, the effect of siloxane is more remarkably exhibited. On the other hand, when the coverage is 95% or less, there is no risk that siloxane not covering the cellulose particle or an aggregated particle are generated to deteriorate the texture, and the biodegradability effect of the cellulose particle can be reliably obtained.

The volume average particle diameter of the composite particle of the present invention is preferably 1 to 300 $\mu$m, more preferably 1 to 150 $\mu$m, and still more preferably 1 to 50 $\mu$m. Here, the volume average particle diameter of the composite particle of the present invention is the 50% cumulative volume particle diameter determined from the data obtained by measurement using a laser diffraction particle size distribution measurement device (for example, "SALD2100" manufactured by SHIMADZU CORPORATION).

Furthermore, the composite particle of the present invention may be alkoxy-modified. The method for alkoxy modification is the method disclosed in, for example, JP 2008-37714 A. Specifically, it is a method of surface modification of silica using an alcohol ($C_nH_{2n+1}OH$) to form an alkoxy group on the surface of silica. The value of n is not particularly limited, but, for example, it may be n < 5.

In the present invention, as a preferred embodiment, alkoxylation is performed by boiling and refluxing an alcohol; the length of the side chain of an organic functional group composed of the alkoxy groups is changed to enhance the water resistance; and the hydrophobic part of the organic functional group is changed through the length of the alcohol side chain to make the silica surface hydrophobic.

In the composite particle of the present invention, silica is adhered to the surface of the cellulose particle. That is, in the composite particle of the present invention, only silica may be adhered to the surface of the cellulose particle. Alternatively, in addition to silica, other components may be adhered to the surface of the cellulose particle. Note that, when other components include a rubber particle, the rubber particle may adversely affect the tactile sensation and the like, which means that fewer rubber particles lead to better tactile sensation and spreadability. Therefore, in the composite particle of the present invention, the content of a rubber particle is preferably less than 0.1 parts by mass. More preferably, no rubber particle is adhered to the surface of the cellulose particle.

In addition, the composite particle of the present invention preferably forms a coating film containing the composite particle and having a coefficient of static friction of less than 0.50. With such a composite particle, it is possible to obtain a cosmetic with excellent slipperiness.

[Cosmetic]

The composite particle of the present invention can be formulated and used in cosmetic, and the blending amount of the composite particle in the cosmetic is preferably 1 to 50 mass% based on the total amount of the cosmetic. When the amount is 1 mass% or more, sufficient effects can be obtained, and when the amount is 50 mass% or less, there are no issues such as the whiteness becoming too noticeable.

As other components in the cosmetic formulation other than the composite particle of the present invention, oil agents, solvents, and powders other than the composite particle of the present invention can be included.

Examples of oil agents include hydrocarbons, silicone oils, triglycerides, ester oils, fats, waxes, higher fatty acids with 12 to 20 carbon atoms, higher alcohols with 8 to 20 carbon atoms, etc., and among these, low-boiling-point silicone oils, low-boiling-point isoparaffin hydrocarbons, triglycerides, and ester oils are preferable to other oil agents. Examples of the low-boiling-point silicone oils include octamethylcyclotetrasiloxane, decamethylcyclopentasiloxane, and tetradecamethylcyclohexasiloxane. Examples of the ester oils include fatty acid esters and glycerin fatty acid esters each having 6 to 20 carbon atoms.

The content of the oil agent in the cosmetic may vary depending on the formulation of the cosmetic, and is preferably 0.1 to 95 mass%, more preferably 1 to 80 mass%, with respect to the total amount of the powder and the composite particle of the present invention. When the content is 0.1 mass% or more, the slipperiness, moisturizing effect, etc., of the oil agent can be sufficiently exhibited, and when the content is 95 mass% or less, the storage stability is favorable.

Examples of the solvents include medium and lower alcohols, aromatic alcohols, etc., and preferred are lower alcohols having 1 to 4 carbon atoms, such as isopropyl alcohol. The content of the solvent in the cosmetic of the present invention may vary depending on the formulation of the cosmetic, and is preferably 0.1 to 80 mass%, more preferably 1 to 50 mass%, with respect to the total amount of the powder and the composite particle of the present invention.

The powder indicates a material that can be used for normal (makeup) cosmetic, and is not particularly limited. Typically, the powder has an average particle diameter of 0.1 to 50 $\mu$m, and is, for example, colorants such as inorganic color pigments, inorganic white pigments, organic pigments, pearlescent agents, extender pigments, organic powders, and the like.

Examples of the powders include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithium mica, silicic acid, anhydrous silicic acid, aluminum silicate, sodium silicate, sodium magnesium silicate, magnesium silicate, magnesium aluminum silicate, calcium silicate, barium silicate, strontium silicate, metal salts of tungstic acid, hydroxyapatite, vermiculite, Higilite, bentonite, montmorillonite, hectorite, zeolite, ceramic powder, dicalcium phosphate, alumina, aluminum hydroxide, boron nitride, and boron nitride. Examples of the organic powders include polyamide powder, polyester powder, polyethylene powder, polypropylene powder, polystyrene powder, polyurethane powder, benzoguanamine powder, polymethylbenzoguanamine powder, polytetrafluoroethylene powder, polymethyl methacrylate powder, cellulose, silk powder, nylon powder, nylon 12, nylon 6, silicone powder, spherical polymethylsilsesquioxane powder, styrene-acrylic acid copolymer, divinylbenzene-styrene copolymer, vinyl resin, urea resin, phenol resin, fluororesin, silicon resin, acrylic resin, melamine resin, epoxy resin, polycarbonate resin, microcrystalline fiber powder, starch powder, and lauroyl lysine. Examples of the surfactant metal salt powder include zinc stearate, aluminum stearate, calcium stearate, magnesium stearate, zinc myristate, magnesium myristate, zinc palmitate, zinc laurate, zinc cetyl phosphate, calcium cetyl phosphate, and sodium zinc cetyl phosphate. Examples of the colored pigments include: inorganic red pigments such as iron red, iron oxide, ferric hydroxide, and iron titanate; inorganic brown pigments such as γ-iron oxide; inorganic yellow pigments such as yellow iron oxide and ocher; inorganic black pigments such as black iron oxide and carbon black; inorganic violet pigments such as manganese violet and cobalt violet; inorganic green pigments such as chromium hydroxide, chromium oxide, cobalt oxide, and cobalt titanate; inorganic blue pigments such as Prussian blue and ultramarine blue; lakes of tar-based dyes such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and lakes of natural colorants such as carminic acid, laccaic acid, kalsamin, brasilin, and crocin. Examples of the pearl pigments include titanium oxide-coated mica, titanium mica, iron oxide-treated titanium mica, bismuth oxychloride, titanium oxide-coated bismuth oxychloride, titanium oxide-coated talc, fish scale foil, and titanium oxide-coated colored mica. Examples of the metallic powder pigments include metal powders such as aluminum, gold, silver, copper, platinum, and stainless steel.

Furthermore, the cosmetic of the present invention may further include, in addition to the above-described components, in accordance with the intended purpose, other components commonly used in cosmetics, such as surfactants, oily components, polymer compounds, gelling agents, alkali agents, polyhydric alcohols, pH adjusters, ultraviolet absorbers, antioxidants, preservatives, anti-inflammatory agents, skin-beautifying agents, and fragrances.

Examples of the cosmetic of the present invention include makeup cosmetics such as foundation, face powder, eyeshadow, eyeliner, eyebrow makeups, blush, lipstick, and nail color; basic cosmetics such as emulsions, creams, lotions, calamine lotions, sunscreen agents, suntan agents, aftershave lotions, preshave lotions, facial packs, anti-acne cosmetics, and essences; hair cosmetics such as shampoos, rinses, conditioners, hair colorants, hair tonics, setting agents, hair growth agents, and permanent wave agents; and other cosmetics such as body powders, deodorants, depilatories, soaps, body shampoos, bath agents, hand soaps, and perfumes. The composite particle of the present invention is preferably used in powder cosmetics such as foundation, face powder, eyeshadow, and eyebrow makeups.

EXAMPLE

Examples and Comparative Examples will be described hereinbelow to explain the present invention in more detail, but the present invention is not limited to the following Examples. In the following Examples, "parts" and "%" represent parts by mass and mass%, respectively.

[Example 1]

100 parts by mass of a spherical cellulose particle (A-1) having an average particle diameter of 7 μm, 830 parts by mass of ion-exchanged water, and 1.6 parts by mass of dodecyltrimethylammonium chloride (cation BB, cationic emulsifier produced by NOF CORPORATION) were charged into a 500 mL reactor and stirred for 30 minutes. After cooling to 5°C, 0.5 parts by mass of 25% aqueous ammonia was added, and 22.5 parts by mass of tetramethoxysilane (B-1) was added over 20 minutes. After aging at 8°C for 1 hour, 22 parts by mass of 25% aqueous ammonia was added, followed by aging for 1 hour. The temperature was then raised to 55°C, and the mixture was aged for 1 hour to obtain a slurry. The slurry was subjected to solid-liquid separation by suction filtration, and the resulting solid was then allowed to stand and dry. Electron microscopy confirmed a composite particle in which silicon dioxide is adhered to the surface of the grainy cellulose particle.

[Example 2]

100 parts by mass of the powder obtained in Example 1 was charged into a 500 mL reactor, and 830 parts by mass of isopropanol (produced by Wako Pure Chemical Industries, Ltd.) was added thereto, and the mixture was then heated to 80°C and subjected to reflux for 10 hours to obtain a slurry. The slurry was subjected to solid-liquid separation by suction filtration, and the resulting solid was then allowed to stand and dry, thereby obtaining a powder having excellent monodispersity and fluidity. When the powder was floated on water and allowed to stand for 5 minutes, the powder remained afloat on the water surface and did not settle in the water. When observed after standing for 10 hours or more, the powder was found to have settled in the water. These results indicate that the powder has short-term water repellency.

[Example 3]

The steps of Example 1 were repeated to obtain a powder except that the spherical cellulose particle with an average particle diameter of 7 $\mu$m was replaced with a spherical cellulose particle (A-2) with an average particle diameter of 15 $\mu$m.

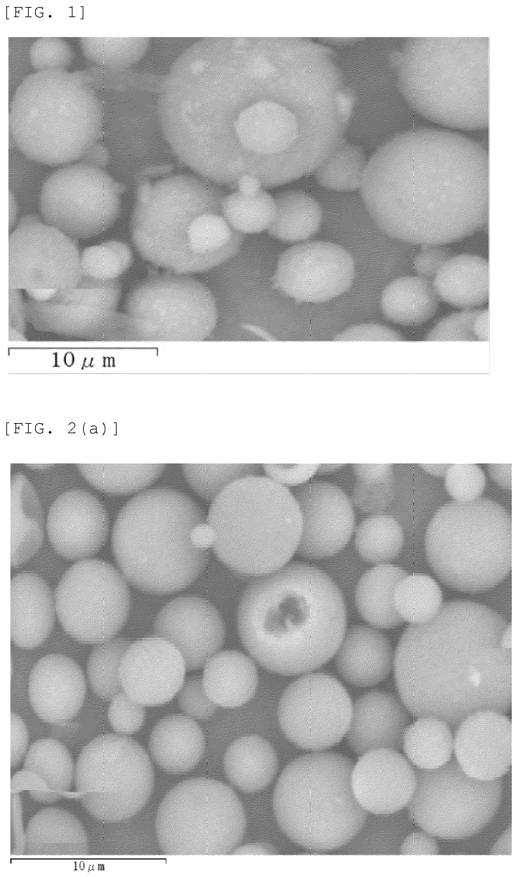

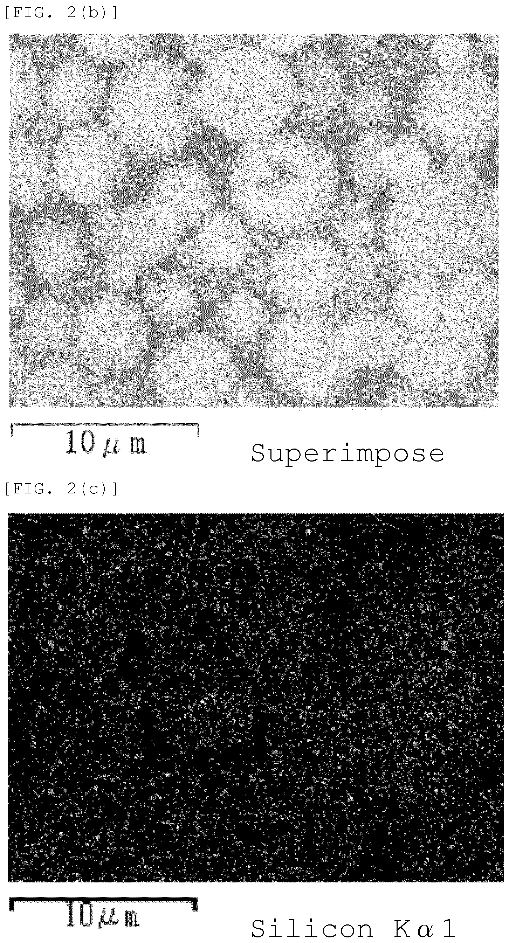

When the composite particle was observed under an electron microscope, particles having a particle diameter of approximately 100 nm were observed on the surface of the particle. Further, when infrared absorption spectrum was measured by means of an FT-IR measurement device (manufactured by SHIMADZU CORPORATION) using a potassium bromide pellet method, characteristic absorption was observed at a wavenumber of 1260 cm$^{-1}$, and 1100 to 1020 cm$^{-1}$. It was thus confirmed that the particle is a composite particle in which silica is adhered to the surface of the grainy cellulose particle. An electron micrograph showing the surface of the above composite particle is shown in FIG. 1. In addition, FIGS. 2(a) to 2(c) show: an electron micrograph (a) of the surface of the above composite particle; a superimposed image (b) including an elemental mapping image of silicon and an elemental mapping image of carbon analyzed by a SwiftED3000 (manufactured by HITACHI LTD.), corresponding to the micrograph (a); and an image (c) displaying only the silicon signal corresponding thereto. Although FIG. 2(b) is slightly unclear due to conversion to a monochrome image, in FIG. 2(b), the white areas substantially represent signals from carbon, and the colored areas substantially represent signals from silicon. These electron microscopic images also confirmed that the composite particle obtained is a particle in which silica is adhered to the surface of the grainy cellulose particle.

[Example 4]

The steps of Example 1 were repeated to obtain a powder except that the cellulose particle of Example 1 was replaced with a spherical cellulose particle (A-3) with an average particle diameter of 5 $\mu$m.

[Comparative Examples 1 to 3]

The cellulose particles (A-1), (A-2), and (A-3) were evaluated without silica treatment.

[Comparative Example 4]

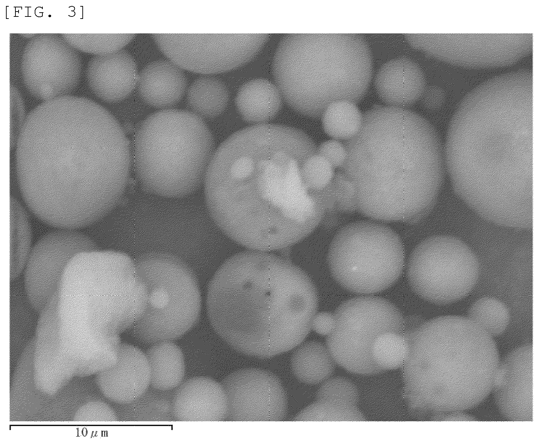

The steps of Example 1 were repeated to obtain a composite particle except that 25% ammonia was not added before and after charging of tetramethoxysilane. The electron micrograph is shown in FIG. 3

[Comparative Example 5]

A spherical silica particle (B-2) was evaluated without any treatment.

The composite particles obtained above were evaluated as follows. The results are shown in Tables 1 and 2.

<Coverage>

Electron micrograph of the surface of 10 composite particles was observed visually to calculate the average coverage. More specifically, in a field of view magnified up to 10,000 times, the area of a region in which silica fine particles are not adhered in the composite particle was measured from individually captured images, and the percentage of the part covered with silica was calculated by:

$$\text{Coverage (\%)} = \text{Covered area/Surface area} \times 100.$$

<Method of Measuring Tactile Sensation and Spreadability>

A suitable amount of powder sample is adhered to the finger and contacted on black carbon paper to slip on the paper at a stroke. The feel during sliding was defined as "tactile sensation", and how long the sliding trace extended was defined as "spreadability", and these properties were evaluated based on the following three levels:

    Good: exhibited a distinctive and favorable slipperiness
    Fair: exhibited a slightly favorable slipperiness
    Poor: felt resistance on the finger and lacked slipperiness

<Method of Measuring Coefficient of Friction>

About 1 g of a powder sample was uniformly spread over a bioskin plate (manufactured by Beaulax Co., Ltd.) to prepare a sample piece. Using a HEIDON TYPE-38 (manufactured by Shinto Scientific Co., Ltd.), the frictional force was measured when an indenter loaded with a 100 g weight was brought into vertical contact with the above sample piece and moved at a speed of 3 cm/min, and the coefficient of friction was calculated from the measured frictional force. An indenter having a contact surface with a diameter of 12 mm and provided with artificial leather supplee on the contact surface with the sample piece was used. Note that the preferred range of coefficients of static and dynamic friction are in the range of 0.01 to 0.50, more preferably 0.01 to 0.45, for both coefficients of static and dynamic friction.

<Contact Angle>

A sample piece was prepared in the same manner as the above coefficient of friction measurement, and a 10 $\mu$m water droplet was placed thereon, and the contact angle was measured ten seconds later using a contact angle meter CA-D model (Kyowa Interface Science Co., Ltd.). The preferred range for water repellency material is a contact angle of 90° or more, and more preferably 95° or more.

<Light Diffusion Intensity (Evaluation of Light Diffusibility)>

5 g of powder sample was charged to 45 g of clear lacquer and dispersed in a dispersing mixer at 3000 rpm for 2 minutes. The dispersion liquid was dropped onto black carbon paper to form a circular area having a diameter of 2 cm, and then coated using a 5 MIL coater to form a coating film. After air drying, three different points on the coating film were measured using a gloss meter, and the average value was adopted. A lower intensity of reflected light at an angle of 85° indicates a more matte appearance and better light diffusibility. The preferred range for light diffusion intensity (85°) is 5 or less.

[Table 1]

|  | Example | | | |
|---|---|---|---|---|
|  | 1 | 2 | 3 | 4 |
| A-1 | 100 | 100 |  |  |
| A-2 |  |  | 100 |  |
| A-3 |  |  |  | 100 |
| B-1 | 22.5 | 22.5 | 22.5 | 22.5 |
| B-2 |  |  |  |  |
| Coverage (%) | 50 | 50 | 70 | 50 |
| Alkoxylation treatment | Without | With | Without | Without |
| Tactile sensation | Smooth | Smooth | Smooth | Smooth |
| Coefficient of static friction | 0.30 | 0.44 | 0.35 | 0.39 |
| Coefficient of dynamic friction | 0.27 | 0.38 | 0.33 | 0.31 |
| Contact angle (°) | 109 | 136 | 98 | 99 |
| Spreadability | Good | Good | Good | Good |
| Light Diffusion Intensity (85°) | 3.6 | 3.3 | 2.8 | 8.0 |

[Table 2]

|  | Comparative Example | | | | |
|---|---|---|---|---|---|
|  | 1 | 2 | 3 | 4 | 5 |
| A-1 | 100 |  |  | 100 |  |
| A-2 |  | 100 |  |  |  |
| A-3 |  |  | 100 |  |  |
| B-1 |  |  |  | 22.5 |  |
| B-2 |  |  |  |  | 100 |

(continued)

| | Comparative Example | | | | |
|---|---|---|---|---|---|
| | 1 | 2 | 3 | 4 | 5 |
| Coverage (%) | 0 | 0 | 0 | 0 | 0 |
| Tactile sensation | Smooth | Smooth | Smooth | Smooth | Rough |
| Coefficient of static friction ($\mu$s) | 0.51 | 0.41 | 0.49 | 0.5 | 0.62 |
| Coefficient of dynamic friction ($\mu$k) | 0.47 | 0.37 | 0.43 | 0.56 | 0.57 |
| Contact angle (°) | 10 | 10 | 15 | 10 | 9 |
| Spreadability | Poor | Poor | Poor | Poor | Poor |
| Light Diffusion Intensity (85°) | 4 | 7.5 | 8.3 | 4 | 7.1 |

A-1: Cellulose particle with an average particle diameter of 7 $\mu$m (bulk density: 0.59 g/cm$^3$, angle of repose: 47°)
A-2: Cellulose particle with an average particle diameter of 15 $\mu$m (bulk density: 0.66 g/cm$^3$, angle of repose: 49°)
A-3: Cellulose particle with an average particle diameter of 5 $\mu$m (bulk density: 0.68 g/cm$^3$, angle of repose: 51°)
B-1: Tetramethoxysilane (KBM04, produced by Shin-Etsu Chemical Co., Ltd.)
B-2: Silica powder (average particle diameter: 4 $\mu$m, SYLOBLOC S-400, produced by Moriroku Co., Ltd.)
(Note that the average particle diameter of each of A-1 to A-3 means average particle diameter (D50), and measured by using a laser diffraction particle size distribution measurement device (LA-950V2, HORIBA, Ltd.) after 1 g of the powder sample is dispersed in water.)

As shown in Table 1, in Examples 1 to 4, which involve the composite particle of the present invention, the surface of the cellulose particle was covered with silica, resulting in good tactile sensation and spreadability, as well as excellent slipperiness and light diffusibility. Furthermore, the composite particle of Example 2, which was subjected to alkoxy modification, exhibited water repellency. In contrast, as shown in Table 2, in Comparative Examples 1 to 3, in which the cellulose particle was used alone, and in Comparative Example 4, in which tetramethoxysilane (B-1) was added but no silica was adhered, sufficient spreadability was not obtained despite exhibiting good tactile sensation. In addition, in Comparative Example 5, in which silica powder was used alone, both tactile sensation and spreadability were poor.

[Example 5]

A foundation was prepared using the composite particle produced in Example 1, in accordance with the formulation shown below.

Foundation

| Component | Blending Amount (%) |
|---|---|
| (1) Cellulose-siloxane composite pparticle of Example 1 | 3.0 |
| (2) Acrylic silicone-treated talc(Note 1) | Balance |
| (3) Acrylic silicone-treated sericite | 10.0 |
| (4) Metal soap-treated mica | 2.0 |
| (5) Synthetic phlogopite | 5.0 |
| (6) Spherical silica powder | 5.0 |
| (7) Silicone-treated fine titaniumoxide particle | 12.5 |
| (8) Silicone-treated red iron oxidde | 0.6 |
| (9) Silicone-treated yellow iron ooxide | 2.0 |
| (10) Silicone-treated black iron ooxide | 0.2 |
| (11) Silicone-treated titanium oxide | 6.0 |
| (12) Diisostearyl malate | 2.0 3.5 |
| (13) Glycerol triisostearate | 0.4 |
| (14) Methylpolysiloxane | 3.5 |

(continued)

| Component | Blending Amount (%) |
|---|---|
| (15) Ultraviolet absorber | 5.0 |
| (16) Preservative | Appropriate amount |
| (17) Fragrance | Appropriate amount |
| Total | 100.0 |

(Note 1) NS Talc JA-46R-3F (produced by KAKUHACHI Co., Ltd.)

[Examples 6 to 8, Comparative Examples 6 to 10]

Foundations were prepared in the same formulation as in Example 5, except that the powder was replaced with the composite particles obtained in Examples 2 to 4 and Comparative Examples 1 to 5.

[Example 9]

A foundation was also prepared in the same formulation as in Example 5, except that the amount of the composite particle used in Example 5 was increased to 20%.

The foundations obtained as described above were evaluated in the following manner. The results are shown in Tables 3 and 4.

[Tactile Sensation, Slipperiness]

A use test was performed with 20 trained panelists, and each item related to the tactile sensation and slipperiness of the cosmetic was evaluated based on the evaluation point criteria described below. Subsequently, the evaluation points given by each panelist were totaled, and evaluated based on the evaluation criteria described below.

(Evaluation Point Criteria)

5 points: excellent
4 points: good
3 points: fair
2 points: poor
1 point: very poor

(Evaluation Criteria)

Excellent: a total score of 80 points or higher
Good: a total score of 60 points or higher but less than 80 points
Fair: a total score of 40 points or higher but less than 60 points
Poor: a total score of less than 40 points

[Table 3]

|  | Example 5 | Example 6 | Example 7 | Example 8 | Example 9 |
|---|---|---|---|---|---|
| Type of composite particle | Example 1 | Example 2 | Example 3 | Example 4 | Example 1 |
| Amount of composite particle in the cosmetic | 3.0% | 3.0% | 3.0% | 3.0% | 20% |
| Tactile sensation | Excellent | Excellent | Excellent | Excellent | Excellent |
| Slipperiness | Excellent | Excellent | Excellent | Excellent | Excellent |

[Table 4]

|  | Comparative Example 6 | Comparative Example 7 | Comparative Example 8 | Comparative Example 9 | Comparative Example 10 |
|---|---|---|---|---|---|
| Type of powder | Comparative Example 1 | Comparative Example 2 | Comparative Example 3 | Comparative Example 4 | Comparative Example 5 |
| Amount of powder in the cosmetic | 3.0% | 3.0% | 3.0% | 3.0% | 3.0% |
| Tactile sensation | Good | Good | Good | Good | Fair |
| Slipperiness | Fair | Fair | Fair | Fair | Poor |

As shown in Tables 3 and 4 above, foundations using a cellulose particle without surface treatment or a silica powder exhibit inferior tactile sensation and slipperiness. In contrast, the cellulose-silica composite particle of the present invention shows superior tactile sensation and slipperiness in the resulting foundation compared to the cellulose particle without surface treatment.

The present description includes the following inventions.

[1]: A composite particle comprising: a particle of cellulose or a cellulose derivative; and silica adhered to a surface of the particle, wherein the silica is a polymerized product of a tetraalkoxysilane in an amount of 1 to 60 parts by mass with respect to 100 parts by mass of the particle.

[2]: The composite particle according to [1], wherein the particle of cellulose or a cellulose derivative has a volume average particle diameter of 1 to 300 μm.

[3]: The composite particle according to [1] or [2], wherein the tetraalkoxysilane is tetramethoxysilane.

[4]: The composite particle according to any one of [1] to [3], wherein a coating film comprising the composite particle has a coefficient of static friction of less than 0.50.

[5]: The composite particle according to any one of [1] to [4], wherein the composite particle is alkoxy-modified.

[6]: A composite particle comprising: a particle of cellulose or a cellulose derivative; and silica adhered to a surface of the particle, wherein the particle is coated with silica in a specific surface area of 50% or more.

[7]: The composite particle according to [6], wherein the particle of cellulose or a cellulose derivative has a volume average particle diameter of 1 to 300 μm.

[8]: The composite particle according to [6] or [7], wherein the silica is a polymerized product of a tetraalkoxysilane.

[9]: The composite particle according to [8], wherein the tetraalkoxysilane is tetramethoxysilane.

[10]: The composite particle according to any one of [6] to [9], wherein a coating film comprising the composite particle has a coefficient of static friction of less than 0.50.

[11]: The composite particle according to any one of [6] to [10], wherein the composite particle is alkoxy-modified.

[12]: A method for producing a composite particle comprising a particle of cellulose or a cellulose derivative and silica adhered to a surface of the particle, the method comprising the step of: (1) in the presence of (A) a particle of cellulose or a cellulose derivative, water, and an alkali, hydrolyzing and condensing 1 to 60 parts by mass of (B) a tetraalkoxysilane with respect to 100 parts by mass of the (A) particle to form silica, and adhering the silica to a surface of the (A) particle to obtain the composite particle.

[13]: The method for producing a composite particle according to [12], further comprising the step of: (2) removing water after the step (1).

[14]: A cosmetic comprising the composite particle according to any one of [1] to [11].

[15]: The cosmetic according to [14], wherein the composite particle is contained in an amount of 1 to 50 mass% based on a total amount of the cosmetic.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

INDUSTRIAL APPLICABILITY

When formulated and used in a cosmetic, the composite particle of the present invention can impart properties such as lubricity, a soft tactile sensation, and water repellency to the cosmetic; and the composite particle of the present invention can be formulated into many cosmetics such as hair cosmetics, makeup cosmetics, and sunscreens. In addition, the composite powder of the present invention and a cosmetic containing the composite powder are expected to exhibit biodegradability.

**Claims**

1. A composite particle comprising: a particle of cellulose or a cellulose derivative; and silica adhered to a surface of the particle,
   wherein the silica is a polymerized product of a tetraalkoxysilane in an amount of 1 to 60 parts by mass with respect to 100 parts by mass of the particle.

2. The composite particle according to claim **1,** wherein the particle of cellulose or a cellulose derivative has a volume average particle diameter of 1 to 300 $\mu$m.

3. The composite particle according to claim 1, wherein the tetraalkoxysilane is tetramethoxysilane.

4. The composite particle according to claim 1, wherein a coating film comprising the composite particle has a coefficient of static friction of less than 0.50.

5. The composite particle according to claim 1, wherein the composite particle is alkoxy-modified.

6. A composite particle comprising: a particle of cellulose or a cellulose derivative; and silica adhered to a surface of the particle,
   wherein the particle is coated with silica in a specific surface area of 50% or more.

7. The composite particle according to claim 6, wherein the particle of cellulose or a cellulose derivative has a volume average particle diameter of 1 to 300 $\mu$m.

8. The composite particle according to claim 6, wherein the silica is a polymerized product of a tetraalkoxysilane.

9. The composite particle according to claim 8, wherein the tetraalkoxysilane is tetramethoxysilane.

10. The composite particle according to claim 6, wherein a coating film comprising the composite particle has a coefficient of static friction of less than 0.50.

11. The composite particle according to claim 6, wherein the composite particle is alkoxy-modified.

12. A method for producing a composite particle comprising a particle of cellulose or a cellulose derivative and silica adhered to a surface of the particle, the method comprising the step of:

    (1) in the presence of (A) a particle of cellulose or a cellulose derivative, water, and an alkali, hydrolyzing and condensing 1 to 60 parts by mass of (B) a tetraalkoxysilane with respect to 100 parts by mass of the (A) particle to form silica, and adhering the silica to a surface of the (A) particle to obtain the composite particle.

13. The method for producing a composite particle according to claim 12, further comprising the step of:
    (2) removing water after the step (1).

14. A cosmetic comprising the composite particle according to any one of claims 1 to 11.

15. The cosmetic according to claim 14, wherein the composite particle is contained in an amount of 1 to 50 mass% based on a total amount of the cosmetic.

[FIG. 1]

10 μ m

[FIG. 2(a)]

10 μ m

[FIG. 2(b)]

10 μm　　Superimpose

[FIG. 2(c)]

10μm　　Silicon Kα1

[FIG. 3]

10 μm

## INTERNATIONAL SEARCH REPORT

| International application No. |
|---|
| **PCT/JP2023/045128** |

### A. CLASSIFICATION OF SUBJECT MATTER

*C08J 3/12*(2006.01)i; *A61K 8/00*(2006.01)i; *A61K 8/25*(2006.01)i; *A61K 8/73*(2006.01)i; *C08G 77/42*(2006.01)i; *C08K 3/36*(2006.01)i; *C08L 1/00*(2006.01)i

FI:   C08J3/12 Z CEP; A61K8/00; A61K8/25; A61K8/73; C08G77/42; C08K3/36; C08L1/00

According to International Patent Classification (IPC) or to both national classification and IPC

### B. FIELDS SEARCHED

Minimum documentation searched (classification system followed by classification symbols)

C08J3/12; A61K8/00; A61K8/25; A61K8/73; C08G77/42; C08K3/36; C08L1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2008-545033 A (M-REAL OYJ) 11 December 2008 (2008-12-11)<br>claims 7-10, paragraphs [0028], [0095] | 6-7 |
| X | JP 2015-86173 A (DAITO KASEI KOGYO KK) 07 May 2015 (2015-05-07)<br>claims 1, 4, paragraphs [0018]-[0019], [0021], [0023] | 6-7, 14-15 |
| A | JP 2016-164249 A (KEY TRADING CO., LTD.) 08 September 2016 (2016-09-08)<br>entire text | 1-15 |
| A | JP 2009-114100 A (OSAKA PREFECTURE) 28 May 2009 (2009-05-28)<br>entire text | 1-15 |
| A | JP 2006-45491 A (ERUBU KK) 16 February 2006 (2006-02-16)<br>entire text | 1-15 |
| A | JP 2018-500320 A (L'OREAL) 11 January 2018 (2018-01-11)<br>entire text | 1-15 |

✓ Further documents are listed in the continuation of Box C.    ✓ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **06 March 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
|---|
| **PCT/JP2023/045128** |

### C. DOCUMENTS CONSIDERED TO BE RELEVANT

| Category* | Citation of document, with indication, where appropriate, of the relevant passages | Relevant to claim No. |
|---|---|---|
| A | WO 2020/246555 A1 (KAO CORPORATION) 10 December 2020 (2020-12-10)<br>entire text | 1-15 |
| P, A | WO 2023/234072 A1 (NISSIN CHEMICAL INDUSTRY CO., LTD.) 07 December 2023 (2023-12-07)<br>entire text | 1-15 |

Form PCT/ISA/210 (second sheet) (July 2022)

## INTERNATIONAL SEARCH REPORT
### Information on patent family members

International application No.

**PCT/JP2023/045128**

| Patent document cited in search report | | | Publication date (day/month/year) | Patent family member(s) | | | Publication date (day/month/year) |
|---|---|---|---|---|---|---|---|
| JP | 2008-545033 | A | 11 December 2008 | US 2009/0126891 A1 claims 18-20, paragraphs [0032], [0086] WO 2007/003697 A1 EP 1899533 A1 CN 101218395 A | | | |
| JP | 2015-86173 | A | 07 May 2015 | (Family: none) | | | |
| JP | 2016-164249 | A | 08 September 2016 | (Family: none) | | | |
| JP | 2009-114100 | A | 28 May 2009 | (Family: none) | | | |
| JP | 2006-45491 | A | 16 February 2006 | US 2006/0039986 A1 entire text CN 1721468 A | | | |
| JP | 2018-500320 | A | 11 January 2018 | US 2017/0319458 A1 entire text WO 2016/098910 A1 EP 3233195 A1 KR 10-2017-0094345 A CN 107205909 A | | | |
| WO | 2020/246555 | A1 | 10 December 2020 | US 2022/0202662 A1 entire text EP 3981381 A1 KR 10-2021-0152587 A CN 113966363 A TW 202112342 A JP 2020-200314 A | | | |
| WO | 2023/234072 | A1 | 07 December 2023 | JP 2023-175309 A | | | |

Form PCT/ISA/210 (patent family annex) (July 2022)

**EP 4 640 749 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07196815 A
- WO 2020188698 A1
- JP 2008037714 A